(19) Europäisches Patentamt
European Patent Office
Office européen des brevets

(11) **EP 4 401 850 B1**

(12) **EUROPEAN PATENT SPECIFICATION**

(45) Date of publication and mention
of the grant of the patent:
**30.07.2025 Bulletin 2025/31**

(21) Application number: **22777432.0**

(22) Date of filing: **30.08.2022**

(51) International Patent Classification (IPC):
***B01D 1/06*** (2006.01)

(52) Cooperative Patent Classification (CPC):
**B01D 1/065**

(86) International application number:
**PCT/US2022/042001**

(87) International publication number:
**WO 2023/043609 (23.03.2023 Gazette 2023/12)**

(54) **FALLING FILM APPARATUS AND METHOD FOR USING**

FALLFILMVORRICHTUNG UND VERFAHREN ZUR VERWENDUNG

APPAREIL À FILM TOMBANT ET SON PROCÉDÉ D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.09.2021 US 202163245906 P**

(43) Date of publication of application:
**24.07.2024 Bulletin 2024/30**

(73) Proprietor: **Dow Global Technologies LLC**
**Midland, MI 48674 (US)**

(72) Inventors:
• **KAMAT, Pritish M.**
**Lake Jackson, Texas 77566 (US)**
• **GILLIS, Paul A.**
**Lake Jackson, Texas 77566 (US)**
• **PUNGANUR, Mohan V.**
**Lake Jackson, Texas 77566 (US)**
• **SANDOVAL, Fermin Alejandro**
**Houston, Texas 77077 (US)**
• **JONES, Robert Norman**
**Freeport, Texas 77541 (US)**
• **ELLIOTT, Joseph B.**
**Houston, Texas 77077 (US)**
• **PARSONS, Thomas J.**
**Midland, Michigan 48667 (US)**

(74) Representative: **Boult Wade Tennant LLP**
**Salisbury Square House**
**8 Salisbury Square**
**London EC4Y 8AP (GB)**

(56) References cited:
**US-A- 3 370 635          US-A- 4 298 041**
**US-A1- 2005 042 042**

**Description**

The present invention relates to falling film apparatus, tube inserts for falling film apparatus, and methods for using a falling film apparatus, including separating a crude mixture of isocyanates in a falling film evaporator.

Falling film apparatus are in widespread industrial use as heat exchangers as e.g. disclosed in US4298041. They are useful as evaporators, for performing separations, for performing crystallizations, as well as in other applications. Falling film apparatus have multiple vertical tubes supported at each end by a tube sheet. The tubes are contained in a shell, through which a heat exchange fluid is circulated into contact with the vertical tubes to provide or remove heat from the tubes. A liquid to be so treated is passed downwardly through the interior of the tubes, where the liquid is heated or cooled, as the case may be.

Optimal operation of a falling film apparatus depends on forming a film of the liquid on all interior surfaces of the tubes. The liquid should wet all internal surfaces of each tube and in addition wet those surfaces uniformly and continuously throughout the operation of the device. Non-uniform distribution of the liquid can cause various problems, including reduced operating efficiencies. Unwanted reactions and fouling are common problems caused by improper liquid distribution in falling film apparatus.

For any liquid, there exists a theoretical minimum flow rate per tube that is necessary to maintain a film on the interior tube surfaces. Practically, it is necessary to operate at several times this theoretical minimum flow rate per tube. This constrains the range of operating rates that can be employed. Using higher flow rates promotes uniform wetting, but creates thicker films and can result in less efficient operation. Fluid passing through the device often must be recirculated one or more times to achieve the desired result, thereby increasing operating costs. Residence times are also increased, which can be problematic when, for example, the liquid is sensitive to time-temperature effects. Design improvements which allow a falling film apparatus to operate at flow rates closer to the theoretical minimum flow rate would be desirable.

This invention is in one aspect a falling film apparatus as defined in claim 1.

The second aspect which concerns the associated process is defined in claim 6.

Figure 1 is a front sectional view of an embodiment of an upper tube sheet, hollow tube and tube insert for use in a falling film apparatus of the invention.
Figure 2 is a schematic of a separation apparatus including a falling film evaporator of the invention.
Figure 3 is a front sectional view of a tube insert for use in the invention.
Figure 4 is a front sectional view of a second embodiment of a tube insert for use in the invention.
Figure 5 is an enlarged sectional view of a circumferential rib of a tube insert for use in the invention.
Figure 6 is an enlarged sectional view of a second embodiment of a circumferential rib of a tube insert for use in the invention.
Figure 7 is a front sectional view of a third embodiment of a tube insert for use in the invention.
Figure 8 is a front sectional view of a fourth embodiment of a tube insert for use in the invention.
Figure 9 is a front sectional view of a fifth embodiment of a tube insert for use in the invention.
Figure 10 is a front sectional view of a sixth embodiment of a tube insert for use in the invention.
Figure 11 is a front sectional view of a precursor for machining into a tube insert for use in the invention.
Figure 12 is a front sectional view of a seventh embodiment of a tube insert for use in the invention.
Figure 13 is a front sectional view of an eighth embodiment of a tube insert for use in the invention.

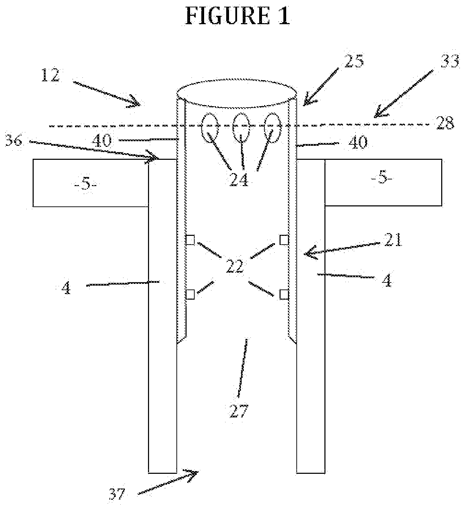

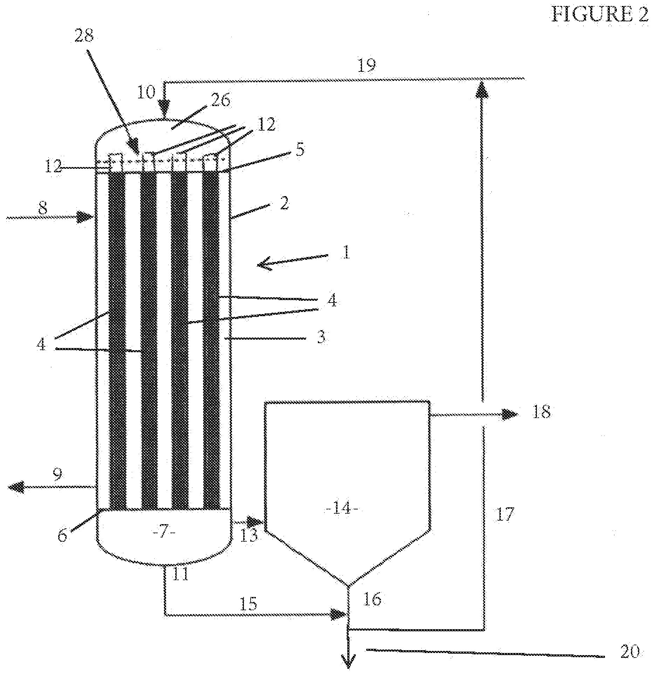

Turning to Figure 1, the invention includes tube sheet 5 and hollow tube 4. Hollow tube 4 has associated tube insert 12. In the cross-sectional view of a simplified apparatus shown in Figure 1, only a single hollow tube 4 is present. More typically, multiple hollow tubes (and associated tube inserts 12) will be present. Each hollow tube 4 has open top end 36 and open bottom end 37. Each hollow tube 4 defines a fluid path from above to below tube sheet 5 through an opening in tube sheet 5 (and to below a lower tube sheet such as lower tube sheet 6 as shown in Figure 2). An opening in tube sheet 5 accommodates each hollow tube 4. Tube sheet 5 is sealed around each hollow tube 4 such that a fluid flowing from above to below tube sheet 5 must pass through a hollow tube 4.

Hollow tubes 4 typically are cylindrical, with a circular cross-section, although hollow tubes 4 can assume other cross-sectional shapes if desired.

Hollow tubes 4 are vertically oriented, by which is it meant hollow tubes are oriented within 3 degrees of vertical, preferably within 1 degree of vertical.

Tube inserts 12 each are located at the top end of the associated hollow tube 4. As shown in Figure 1, tube inserts 12 have an upper section 25 that resides above the upper surface of upper tube sheet 5 and a lower section 21 that extends into the associated hollow tube 4. Sidewall 40 defines an interior flow path through each tube insert 12.

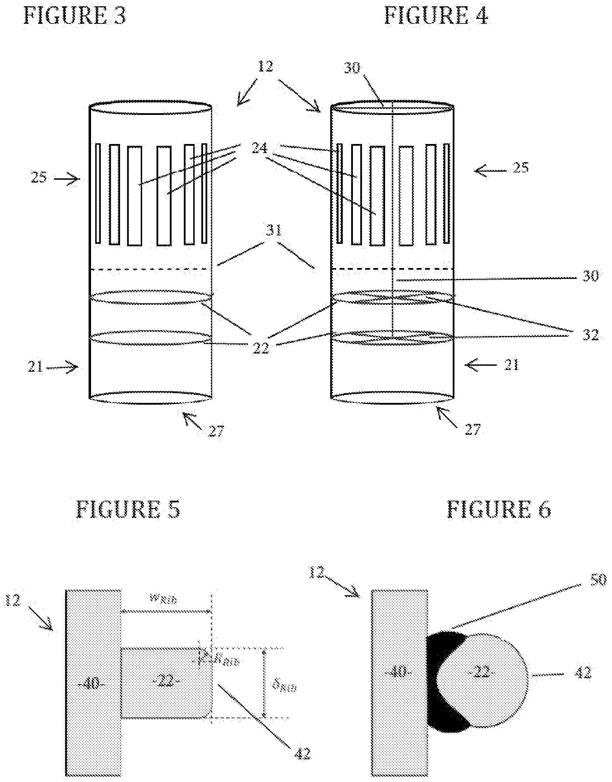

Lower section 21 has outer cross-sectional dimensions equal to or smaller than the inside cross-sectional dimension of the associated hollow tube 4, so lower section 21 can extend into associated hollow tube 4. Lower section 21 may (as shown in Figure 1) or may not extend below the level of tube sheet 5. In Figures 3, 4 and 7-10, dotted lines 31 indicate various possible locations of tube sheet 5 when tube inserts 12 are in place in the falling film apparatus.

Tube inserts 12 each have one or more process fluid openings 24 in upper section 25 for admitting a process fluid into the tube insert. "Process fluid" means any fluid or mixture of fluids (which may further contain a dispersed solid phase) that is passed through tube inserts 12 and hollow tubes 4, including a starting feed fluid introduced into tube inserts 12 and hollow tubes 4 as well as evaporated materials, reaction product or other products produced within hollow tubes. The process fluid contains at least one component that is liquid under the conditions that exist at the locations of circumferential ribs 22 of each tube insert 12. The composition of the process fluid may change as it travels through tube insert 12 and/or hollow tube 4.

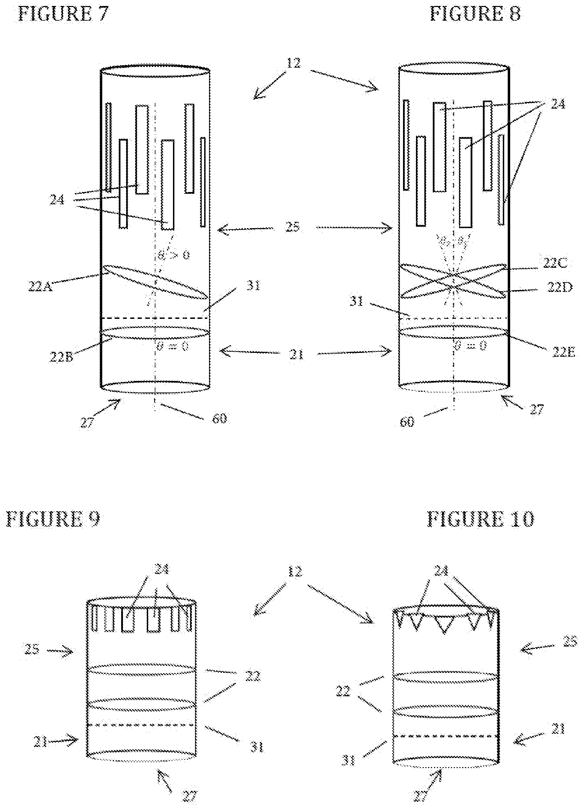

In one embodiment, the process fluid opening is simply the open upper end of tube insert 12. In other embodiments, multiple process fluid openings 24 are provided in the wall of upper section 25 of tube insert 12, as shown in each of Figures 1, 3, 4, 7-10, 12 and 13. The size and shape of the process fluid openings may vary. Elliptical process fluid openings are shown in Figure 1, being arranged about the circumference of upper section 25 of tube insert 12, and displaced longitudinally from the top edge of tube insert 12. In the embodiments shown in Figures 3, 4, 7 and 8, process fluid openings 24 are rectangular slots oriented longitudinally about the circumference of upper section 25 of tube insert 12, and are displaced longitudinally from the top edge of tube insert 12. In the embodiments shown in Figures 9 and 10, process fluid openings 24 take the form of triangular (Fig. 9) or rectangular (Fig. 10) notches arranged about the circumference of the top edge of tube insert 12.

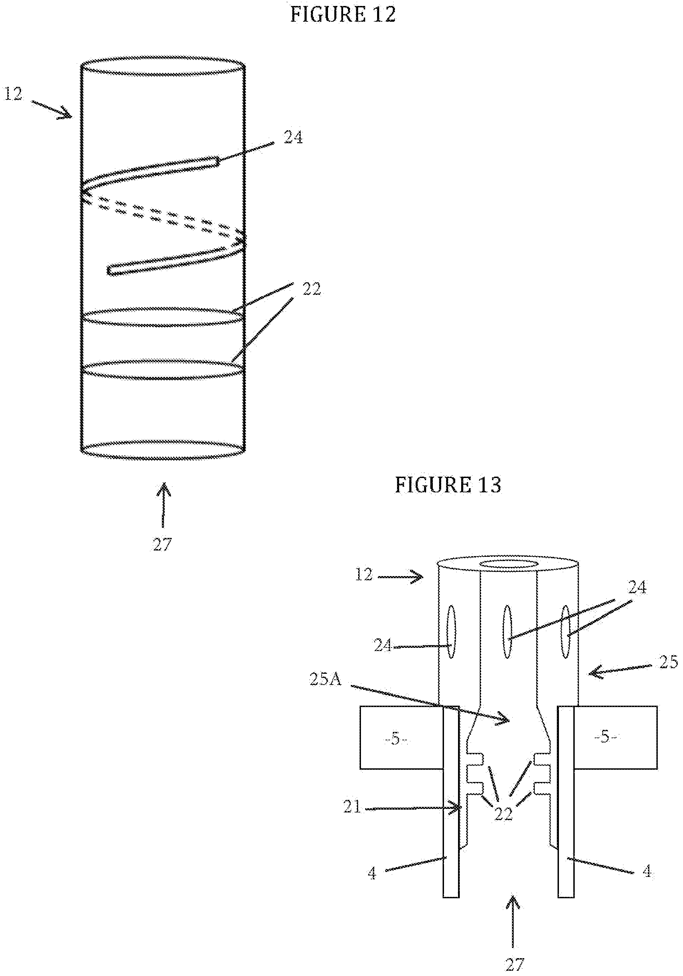

In the embodiment shown in Figure 12, process fluid opening 24 takes the form of helical slots that, as shown, are displaced longitudinally from the top edge of tube insert 12. Although only a single process fluid opening 24 is shown in Figure 12, multiple helical slots preferably are present and are arranged about the circumference of the top edge of tube insert 12 so as to provide overlap between fluid streams entering tube insert 12 from the multiple helical slots. Fluid feed openings 24 can have other shapes as may be convenient or beneficial, such as squares, circles, rhombuses, ellipses, semi-circles, trapezoids, other polygons, or other arbitrary shape. The number of process fluid openings 24 provided in the wall of upper section 25 is not critical to the invention and may vary widely, such as from 2 to 20 or more. Similarly, the size of the multiple process fluid openings may vary considerably as may be necessary or desirable, and the multiple process fluid openings may or may not all be the same size.

Tube inserts 12 also each have an open bottom end 27 (Figures 1, 3, 4, 7-10, 12 and 13) in lower section 21 for discharging process fluids from each tube insert 12 into the associated hollow tube 4. Tube inserts 12 may be chamfered at open bottom end 27, as shown in Figure 1, to produce a smooth transition from tube insert 12 into hollow tube 4.

Tube inserts 12 have one or more circumferential ribs 22 residing on the interior surface of tube inserts 12, below the process fluid opening(s). Circumferential ribs 22 may reside in upper section 25 of tube insert 12 (as is the case in Figures 9 and 10 and for circumferential ribs 22A, 22C and 22D in Figures 7 and 8, respectively), in lower section 21 (as is the case in Figures 1, 3, 4, and for circumferential ribs 22B and 22E in Figures 7 and 8, respectively) and/or at the boundary between upper section 25 and lower section 21 (*i.e.,* a the level of upper tube sheet 5). As shown in Figures 7 and 8, circumferential ribs may reside in both upper section 25 and lower section 21.

Any arbitrary number of circumferential ribs may be provided. For example, the number of circumferential ribs is up to 10, up to 5 or up to 3, and only a single circumferential rib may be present.

By "circumferential", it is meant that the rib extends entirely around the interior wall of tube insert 12, forming a circle (if oriented perpendicular to longitudinal central axis 60 of tube insert 12, as shown in Figures 3, 4, 9, 10, 12 and 13) or an ellipse (if oriented non-perpendicularly to longitudinal central axis 60 of tube insert 12, as illustrated by circumferential ribs 22A, 22C and 22D in Figures 7 and 8. As shown in Figures 7 and 8, multiple circumferential ribs may be oriented at different angles to longitudinal central axis 60 if desired. In Figures 7 and 8, circumferential ribs 22A, 22C and 22D are oriented non-perpendicularly to longitudinal central axis 60 whereas circumferential ribs 22B and 22E are oriented perpendicularly to longitudinal central axis 60. All circumferential ribs 22 may be oriented at the same angle to longitudinal central axis 60.

Each circumferential rib 22 projects radially inward (*i.e.,* toward the central longitudinal axis) from the interior surface of tube insert 12. The radial width $W_{Rib}$ (see Fig. 5) of each circumferential rib in some embodiments is at least 0.25 mm, and in some embodiments is at least 1 mm or at least 2.5 mm. The radial width $W_{Rib}$ may be, for example, as great as one-eighth the inner diameter $ID_{TI}$ of tube insert 12 ($ID_{TI}/8$), and in particular embodiments may be up to $ID_{TI}/12$ or $ID_{TI}/16$.

Each circumferential rib 22 has a longitudinal width $\delta_{Rib}$ (Figure 5). $\delta_{Rib}$ in some embodiments is at least 0.25 mm, and in some embodiments is at least 1 mm or at least 2.5 mm. $\delta_{Rib}$ may be, for example, up to 25 mm, up to 10 mm, up to 7.5 mm or up to 5 mm.

The inward edges 42 of circumferential ribs 22 may be curved, and when curved the inward edges 42 may have a radius of curvature $R_{Rib}$ such that $R_{Rib} \leq \delta_{Rib}/2$. When $R_{Rib} = \delta_{Rib}/2$ the inward edge 42 will have a semi-circular cross-section. $R_{Rib}$ may be $\leq \delta_{Rib}/4$, $\leq \delta_{Rib}/8$, or $\leq \delta_{Rib}/12$ and in some embodiments $R_{Rib} > \delta_{Rib}/32$, preferably $> \delta_{Rib}/24$ or $> \delta_{Rib}/16$.

Circumferential ribs 22 may be integral with sidewall 40 of tube insert 12, or may be a separately-produced member that is affixed into position within tube insert 12. If separately-produced, circumferential ribs 22 may be affixed to sidewall 40 by, for example, welding or gluing, such as weld or adhesive 50 (Figure 6), or mechanically through the use of various fasteners, and hangers such as hanger apparatus 30 and supports 32 (Figure 4). Circumferential slots for receiving circumferential ribs 22 may be provided in sidewall 40.

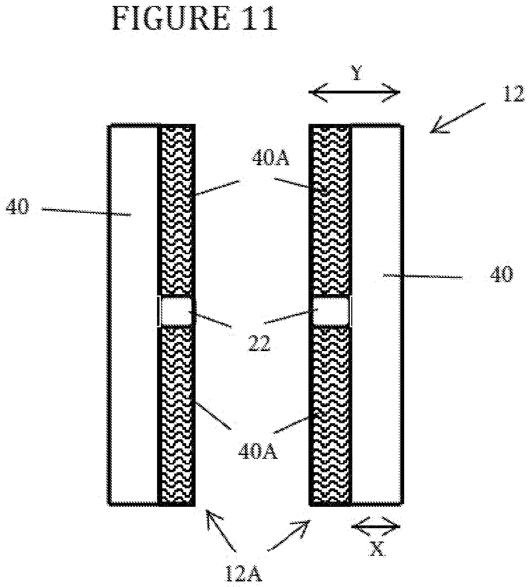

Integral circumferential ribs 22 may be produced by machining the interior of sidewall 40 of precursor tube 12A to remove wall material above and below the location of each circumferential rib 22, as shown in Figure 11. Tube insert 12 is fabricated from a precursor tube 12A, which has a wall thickness Y greater than sidewall thickness X of tube insert 12 made therefrom. Shaded portions 40A of sidewall 40 in Figure 11 are removed by machining to produce tube insert 12 having a sidewall thickness X and circumferential rib 22. The combined thickness of the wall and rib may be equal to original wall thickness Y.

The interior surface of sidewall 40 may be of uniform interior diameter (except for the ribs). In embodiments such as shown in Figure 13, the interior surface of sidewall 40 in upper section 25 of tube insert 12 tapers outwardly in a region 25A starting below fluid process openings 24 to above ribs 22, thereby increasing the interior diameter of tube insert 12 through that region 25A of upper section 25. Such a design further facilitates smooth annular flow of the process fluids towards circumferential ribs 22.

In operation, a process fluid is introduced onto the upper surface of tube sheet 5 and upon reaching a certain height (such as indicated by liquid interface 28 in Figures 1 and 2) spills over into upper section 25 of tube inserts 12 through process fluid openings 24. Upon entering tube inserts 12, the process fluids are driven downward within the associated tube inserts 12 past circumferential ribs 22 by gravitational and/or differential pressure forces, continuing downward through lower section 21 of tube inserts 12, then out of tube inserts 12 through open bottom ends 27 and into hollow tubes 4. Circumferential ribs 22 capture the momentum of the falling process fluid to distribute the fluid evenly about the circumference of tube insert 12, forming a uniform film without dry spots. The uniform film is maintained as the process fluid passes through open bottom ends 27 and into and through associated hollow tubes 4.

Tube inserts 12 may further include one or more features such as flow deflectors that produce a tangential flow of process fluid entering the tube insert. The process fluid openings may be machined to produce a tangential flow of process fluid entering the tube insert.

Turning to Figure 2, falling film apparatus 1 includes outer shell 2, which defines a vessel having an enclosed interior volume. Upper tube sheet 5 and lower tube sheet 6 partition the enclosed interior volume into upper portion 26 residing above upper tube sheet 5, middle portion 3 residing between upper tube sheet 5 and lower tube sheet 6, and lower portion 7 residing below lower tube sheet 6. Hollow tubes 4 define fluid paths from upper portion 26 through middle portion 3 to lower portion 7 of the enclosed interior volume. An opening in upper tube sheet 5 and an opening in lower tube sheet 6 accommodates each hollow tube 4. Upper tube sheet 5 and lower tube sheet 6 each are sealed around each hollow tube 4 such that a fluid flowing from upper portion 26 to below lower portion 7 must pass through a hollow tube 4.

Falling film heat apparatus 1 further includes at least one process fluid inlet port 10 for introducing a process fluid into upper portion 26 of the interior space enclosed by shell 2. Multiple process fluid inlet ports 10 may be provided. Falling film apparatus 1 further includes at least one process fluid outlet port 11 for removing process fluid(s) from lower portion 7 of the interior space enclosed by shell 2. Multiple process fluid outlet ports 11 may be provided. In the embodiment shown in Figure 1, a separate gas outlet port 13 is provided for removing vapor from lower portion 7 of the space enclosed by shell 2.

Falling film apparatus 1 further includes at least one heat exchange fluid inlet port 8 for introducing a heat exchange fluid into interior space 3 and at least one heat exchange fluid outlet port 9 for removing heat exchange fluid from interior space 3.

In addition to the foregoing features, falling film apparatus 1 may include various optional components. A distributor may be provided to distribute process fluid onto upper tube sheet 5. A wide variety of distribution systems are available to ensure the uniformity of the liquid level on the top tube sheet. One type of distributor is a flat-bottomed container installed above upper tube sheet 5. The container has holes that allow the process fluid to flow onto upper tube sheet 5 between tube inserts 12. A spray distribution system sprays droplets of process fluid over upper tube sheet 5 and/or a flat-bottomed container installed above upper tube sheet 5. Other useful distributors include, for example, any of those described in US Patent Nos. 4,154,642, 4,199,537 and 9,101,852, and US Published Patent Application No. 2020/0030712. Other optional components include various valves, pumps, automated process control devices, and the like.

In operation, a process fluid is introduced into upper portion 26 via one or more inlet ports 10. The process fluid pools on the upper surface of upper tube sheet 5 and upon reaching a certain height (reference numeral 28 in Figs. 1 and 2) spills over into upper section 25 of tube inserts 12 through process fluid openings 24. Upon entering tube inserts 12, the process fluid falls under force of gravity and/or applied pressure downwardly past circumferential ribs 22, through lower section 21 of tube inserts 12, then out of tube inserts 12 through open bottom ends 27 and into and through hollow tubes 4, ultimately passing into lower portion 7 of heat exchange apparatus 1. As described before, the falling process fluid upon passing circumferential ribs 22 becomes distributed evenly about the circumference of tube insert 12, forming a uniform film without dry spots. The uniform film is maintained as the process fluid passes through open bottom ends 27 and into and through associated hollow tubes 4.

In heat exchange operations, hollow tubes 4 are maintained at a different temperature than the process fluid introduced via feed inlet port(s) 10, and thus heat is exchanged between hollow tubes 4 and the process fluid. The hollow tube temperature may be higher or lower than that of the incoming process fluid. The hollow tube temperature typically is higher for operations such as evaporations, pasteurizations and performing chemical reactions, and lower for operations such as crystallizations.

Heat is supplied or removed from hollow tubes 4 via a heat exchange fluid that is introduced into middle portion 3 via heat exchange fluid inlet port 8. The heat exchange fluid circulates within interior space 3, between upper tube sheet 5 and lower tube sheet 6 and in contact with hollow tubes 4, heating or cooling hollow tubes 4 (as the case may be), and is withdrawn through heat exchange fluid outlet port 9. The heat exchange fluid may be a liquid and/or a gas. Some or all of the heat exchange fluid may undergo a phase change within the vessel; steam, for example, may partially or entirely condense within the vessel. Heat exchange fluids are selected at least partially with the desired operating temperatures in mind. Examples of other heat exchange fluids include liquid water, air, nitrogen, argon, helium, liquid and/or gaseous halocarbons (including hydrohalocarbons), silicone fluids, ethylene glycol, propylene glycol and other alkylene glycols and polyalkylene glycols, various alkylated aromatic compounds, various polyester compounds, and the like.

In some embodiments, the falling film apparatus of the invention is used to perform evaporations. In such embodiments, the process fluid is a single-component liquid, which is to be evaporated within hollow tubes 4 or, more typically, a multicomponent fluid containing at least one component that is to be separated from at least one other component by fractional distillation within hollow tubes 4. Evaporations typically produce one or more gaseous products representing component(s) of the process fluid that become evaporated within hollow tubes 4, and one or more liquid products which are components of the process fluid that pass through hollow tubes 4 without evaporating. It is generally preferred to establish a flow of gases downward through hollow tubes 4 so that gaseous products are removed from the bottom of hollow tubes 4; however it is within the scope of the invention to remove gaseous products from the top of hollow tubes 4.

The falling film apparatus shown in Figure 2 is particularly adapted for performing evaporations. The annular film of process fluid flowing downwardly into and through hollow tubes 4 is heated in hollow tubes 4, and at least a portion of one or more components of the process is volatilized to form a gas.

In the embodiment shown in Figure 2, both the volatilized (gaseous) and non-volatilized components of the process fluid flow into lower portion 7 of the vessel, where they are separated. In the embodiment shown, volatilized component(s) are removed from lower portion 7 via line 13, through which they are transferred to optional gas-liquid separator 14 to remove entrained non-volatilized material from the gaseous products. The gaseous products are then recovered via line 18.

Non-volatilized component(s) of the process fluid are removed from lower portion 7 of the vessel via outlet port 11 and line 15. In the optional arrangement shown, non-volatilized component(s) removed via line 15 are combined with additional quantities of non-volatilized component(s) removed from gas-liquid separator 14 via line 16. A non-volatilized product stream is withdrawn via recovery line 20.

All or a portion of the non-volatilized components recovered from falling film apparatus 1 may be recycled back into falling film apparatus 1 if desired, as is the case, for example, of incomplete evaporation of volatile components of the process fluid. In Figure 1, for example, a recycle stream is taken via line 17, recombined with fresh process fluid, and the mixture fed into upper portion 26 of enclosed interior space 3 via line 19 and inlet port 10.

An important advantage of this invention is that annular films of liquid components of the process fluid can be formed without dry spots on all internal surfaces of tube inserts 12 and hollow tubes 4, even at low liquid flow rates. Lower liquid flow rates produce thinner films. Thinner films allow for faster and more uniform heating or cooling of the process fluid and, in the case of evaporations, more complete removal of volatile components from the process fluid. As a result, less material needs to be recycled. Recycled material experiences a more severe thermal history than material processed in a single pass, as the recycled material is exposed to the elevated temperatures for a much longer time period. When the recycled material contains heat-sensitive components, the ability to reduce recycling, thereby reducing exposure times to elevated temperatures, is often a significant advantage.

The ability to produce annular films without dry spots at low flow rates also extends the range of conditions at which the falling film apparatus can be operated. For example, it may be desirable or necessary to operate at relatively low flow rates at certain times, such as during start-ups or shutdowns, without fouling the apparatus or producing non-prime material. The falling film apparatus of the invention permits operation over a wide range of flow rates.

Flow rates though a hollow tube apparatus can be expressed in terms of a minimum wetting rate $\Gamma_{min}$, which is a function of the contact angle $\theta$ (between the entering process fluid and the tube or insert), and the surface tension $\sigma$, viscosity $\mu$, gravitational constant g and density $\rho$ of the process fluid, as follows:

$$\Gamma_{min} = 1.69\left(\frac{\mu\rho}{g}\right)^{1/5}(\sigma(1-\cos\theta))^{3/5}$$

<div align="right">(Equation 1)</div>

Previous falling film apparatus with tube inserts typically operate at a flow rate of 5 or more times $\Gamma_{min}$; they do not produce uniform annular films at lower flow rates. By contrast, the falling film apparatus of this invention operates well at flow rates as low as 1.5 $\Gamma_{min}$ or even lower, and also operate well at much greater flow rates. Thus, in some embodiment of the invention, the falling film apparatus of the invention is operated at a flow rate of 1.5 $\Gamma_{min}$ to 10 $\Gamma_{min}$, 1.5 $\Gamma_{min}$ to 5.0 $\Gamma_{min}$ or 1.5 to 3 $\Gamma_{min}$.

The falling film apparatus of the invention is useful for performing many types of separations, including producing concentrated foods such as concentrated fruit juices and evaporated and/or condensed milk, manufacturing alcoholic beverages such as whiskeys, as well as in many chemical and/or petrochemical processes.

Among the many chemical separations for which the falling film apparatus is useful is the separation of a crude isocyanate mixture produced by phosgenating a mixture of methylene dianiline with higher polymethylene polyanilines. In such a separation, diphenyl methylene diisocyanate (MDI) is separated from higher polymethylene polyphenylene polyisocyanates (having three or more phenyl isocyanate groups) by passing the crude mixture through the falling film apparatus operated at a tube temperature sufficient to volatilize the MDI but not the higher polymethylene polyphenylene polyisocyanates. This produces an MDI-rich vapor stream that may contain, for example, at least 98% by weight MDI, and a liquid stream of polymethylene polyphenylene polyisocyanates that is, relative to the starting crude mixture, enriched in polymethylene polyphenylene polyisocyanate and depleted in MDI.

**Comparative Sample A**

A hollow stainless steel tube having an inner diameter of 4.47 cm is fitted in a tube sheet positioned in the bottom surface of a container. A tube insert having an outer diameter of 4.47 cm and wall thickness of 0.018 cm is inserted into the hollow tube such that an upper section resides above the level of the tube sheet and a lower section resides within the hollow tube below the level of the tube sheet. Eight process fluid openings in the form of longitudinal slots 3 cm in width are spaced evenly about the circumference of the upper section of the tube insert. Associated with each of the process fluid openings is a flow deflector positioned at an angle of about 20 degrees to the open face of the slot. The flow deflectors produce a tangential flow entry of the process fluid into the tube insert. The interior surface of the tube insert below the process fluid openings is smooth and of a constant diameter.

A fluid having (at the temperature of the experiment) a density of 1106 kg/m$^3$, a viscosity of 1.9 cP, a surface tension of 31 mN/m and an advancing contact angle with stainless steel of 60 degrees is poured into the container to create a flow rate of 82 kg/h, yielding a wetting rate $\Gamma$ of 0.162 kg/m-s. The minimum wetting rate $\Gamma_{min}$ for this fluid is 0.099 kg/m-s, as calculated according to Equation 1 above. The operating ratio $\Gamma/\Gamma_{min}$ is 1.64. Under these conditions, the liquid forms rivulets on the inner walls of the tube insert and does not uniformly wet the walls. A uniform annular film is produced only when the flow rate is increased to produce an operating ratio $\Gamma/\Gamma_{min}$ of greater than 4.

**Example 1**

Comparative Sample A is repeated, except this time the tube insert has 2 circumferential ribs in the inner wall beneath the process fluid openings. $R_{Rib}$ is 0.762 mm, $W_{Rib}$ is 2.03 mm, and $\delta_{Rib}$ is 1.52 mm. A uniform film forms as the process fluid passes downwardly past the circumferential ribs, wetting all interior surfaces of the tube insert, at the operating ratio $\Gamma/\Gamma_{min}$ = 1.64.

**Example 2**

Comparative Sample A is again repeated, except this time the tube insert lacks the flow deflectors and there are three circumferential ribs. The circumferential ribs have the same dimensions as those described in Example 1. At the operating ratio $\Gamma/\Gamma_{min}$ = 1.64, a uniform film forms as the process fluid passes downwardly past the circumferential ribs, even without the benefit of tangential fluid entry. All interior surfaces of the tube insert become wetted.

**Claims**

1.   A falling film apparatus comprising:

(a) an outer shell (2) enclosing an interior volume,

(b) upper (5) and lower (6) tube sheets partitioning the interior volume into separate upper (26), middle (3) and lower (7) chambers,

(c) one or more vertically oriented hollow tubes (4) having open top and bottom ends, the hollow tubes each defining a fluid path from the upper chamber (26) to the lower chamber (7),

(d) for each at least one vertically oriented hollow tube (4), an associated tube insert (12) located at the top end of the associated hollow tube, the tube insert (12) comprising a hollow member having (i) an upper section (25) residing above the upper tube sheet (5), (ii) a lower section extending into the associated hollow tube (4), (iii) one or more process fluid openings (24) in the upper section (25) for admitting a process fluid into the tube insert (12), (iv) an open bottom end for transferring a thin film of the process fluid from an interior surface of the tube insert (12) onto an interior surface of the associated hollow tube (4); and (v) one or more circumferential ribs (22) residing on the interior surface of the tube insert (12) below the one or more process fluid opening(s) (24);

(e) at least one process fluid inlet port (10) for introducing a process liquid into the upper chamber (26) and into contact with an upper surface of the upper tube sheet (5),

(f) at least one process fluid outlet port (11) for removing at least treated process fluid from the lower chamber (7);

(g) at least one heat exchange fluid inlet port (8) for introducing a heat exchange fluid into the middle chamber (3); and

(h) at least one heat exchange fluid outlet port (9) for removing the heat exchange fluid from the middle chamber (3).

2. The falling film apparatus of claim 1 wherein the at least one vertically oriented hollow tube(s) (4) and associated tube inserts (12) each have circular cross-sections, each circumferential rib (22) has a radial width $W_{Rib}$ of at least 0.25 mm and at most $ID_I/8$, wherein $ID_I$ represents an inner diameter of the tube insert (12), and each circumferential rib (22) has curved inward edges (42) having a radius of curvature $R_{Rib} < \delta_{Rib}/2$, wherein $\delta_{Rib}$ represents the longitudinal thickness of the circumferential rib (22).

3. The falling film apparatus of claim 1 or 2 wherein the upper section of the tube insert (12) is adapted to produce a tangential flow of process fluid into the tube insert (12).

4. The falling film apparatus of any preceding claim wherein the one or more process fluid openings (24) in the upper section of the tube sheet (12) are helical slots.

5. The falling film apparatus of any preceding claim further comprising a process fluid distributor above the upper tube sheet.

6. A process for separating components of a liquid mixture, comprising introducing a process fluid containing the liquid mixture into a falling film apparatus of any preceding claim, flowing the process fluid through the tube insert(s) (12) of the falling film to form an annular film of the process fluid, then transferring the film of the process fluid onto interior surfaces of associated heated hollow tube(s) (4) of the falling film apparatus and flowing the film downwardly through the heated hollow tube(s) (4), whereby at least one component of the liquid mixture is at least partially vaporized from the film as the film flows downwardly through the heated hollow tube(s) (4), and at least one component of the liquid mixture is not volatilized.

7. The process of claim 6, wherein the starting process fluid contains diphenylmethane diisocyanate and polymethylene polyphenylene polyisocyanates and diphenylmethane diisocyanate is at least partially vaporized from the film as the film flows downwardly through the heated hollow tube(s) (4) to produce a vapor stream containing diphenylmethane diisocyanate and a liquid stream that is, relative to the starting process fluid, enriched in polymethylene polyphenylene polyisocyanates and depleted in diphenylmethane diisocyanate.

**Patentansprüche**

1. Fallfilmeinrichtung, umfassend:

(a) eine Außenhülle (2), die ein Innenvolumen umschließt,

(b) einen oberen (5) und einen unteren (6) Rohrboden, die das Innenvolumen in eine getrennte obere (26), mittlere (3) und untere (7) Kammer unterteilen,

(c) ein oder mehrere vertikal ausgerichtete Hohlrohre (4), die offene oberseitige und unterseitige Enden aufweisen, wobei die Hohlrohre jeweils einen Fluidweg von der oberen Kammer (26) zu der unteren Kammer (7) definieren,

(d) für jedes mindestens eine vertikal ausgerichtete Hohlrohr (4) einen zugehörigen Rohreinsatz (12), der sich an dem oberseitigen Ende des zugehörigen Hohlrohrs befindet, der Rohreinsatz (12) umfassend ein hohles Element, das aufweist (i) einen oberen Abschnitt (25), der über dem oberen Rohrboden (5) liegt, (ii) einen unteren Abschnitt, der sich in das zugehörige Hohlrohr (4) erstreckt, (iii) eine oder mehrere Prozessfluidöffnungen (24) in dem oberen Abschnitt (25) zum Einlassen eines Prozessfluids in den Rohreinsatz (12), (iv) ein offenes unterseitiges Ende zum Übertragen eines Dünnfilms des Prozessfluids von einer Innenoberfläche des Rohreinsatzes (12) auf eine Innenoberfläche des zugehörigen Hohlrohrs (4); und (v) eine oder mehrere Umfangsrippen (22), die auf der Innenoberfläche des Rohreinsatzes (12) unterhalb der einen oder der mehreren Prozessfluidöffnung(en) (24) liegen;

(e) mindestens ein Prozessfluideinlasskanal (10) zum Einbringen einer Prozessflüssigkeit in die obere Kammer (26) und in Kontakt mit einer oberen Oberfläche des oberen Rohrbodens (5),

(f) mindestens ein Prozessfluidauslasskanal (11) zum Entfernen mindestens behandelten Prozessfluids aus der unteren Kammer (7);

(g) mindestens ein Wärmeaustauschfluideinlasskanal (8) zum Einbringen eines Wärmeaustauschfluids in die mittlere Kammer (3); und

(h) mindestens ein Wärmeaustauschfluidauslasskanal (9) zum Entfernen des Wärmeaustauschfluids aus der mittleren Kammer (3).

2. Fallfilmeinrichtung nach Anspruch 1, wobei das mindestens eine oder die mehreren vertikal ausgerichteten Hohlrohre (4) und die zugehörigen Rohreinsätze (12) jeweils einen kreisförmigen Querschnitt aufweisen, jede Umfangsrippe (22) eine radiale Breite $W_{Rippe}$ von mindestens 0,25 mm und höchstens $ID_I/8$ aufweist, wobei $ID_I$ einen Innendurchmesser des Rohreinsatzes (12) darstellt, und jede Umfangsrippe (22) gekrümmte nach innen zeigende Kanten (42) aufweist, die einen Krümmungsradius $R_{Rippe} < \delta_{Rippe}/2$, aufweisen, wobei $\delta_{Rippe}$ die Längsdicke der Umfangsrippe (22) darstellt.

3. Fallfilmeinrichtung nach Anspruch 1 oder 2, wobei der obere Abschnitt des Rohreinsatzes (12) angepasst ist, um einen tangentialen Fluss von Prozessfluid in den Rohreinsatz (12) zu erzeugen.

4. Fallfilmeinrichtung nach einem der vorstehenden Ansprüche, wobei die eine oder die mehreren Prozessfluidöffnungen (24) in dem oberen Abschnitt des Rohrbodens (12) spiralförmige Schlitze sind.

5. Fallfilmeinrichtung nach einem der vorstehenden Ansprüche, ferner umfassend einen Prozessfluidverteiler über dem oberen Rohrboden.

6. Verfahren zum Trennen von Komponenten eines Flüssigkeitsgemischs, umfassend das Einbringen eines Prozessfluids, das Flüssigkeitsgemisch enthält, in eine Fallfilmeinrichtung nach einem der vorstehenden Ansprüche, das Fließenlassen des Prozessfluids durch den Rohreinsatz/die Rohreinsätze (12) des Fallfilms, um einen ringförmigen Film des Prozessfluids zu bilden, das anschließende Übertragen des Films des Prozessfluids auf Innenoberflächen (eines) zugehörigen/r erwärmter/erwärmten Hohlrohre(s) (4) der Fallfilmeinrichtung und das abwärts Fließenlassen des Films durch das/die erwärmte(n) Hohlrohr(e) (4), wobei mindestens eine Komponente des Flüssigkeitsgemischs mindestens teilweise aus dem Film verdampft wird, während der Film durch das/die erwärmte(n) Hohlrohr(e) (4) abwärts fließt, und mindestens eine Komponente des Flüssigkeitsgemischs nicht verflüchtigt wird.

7. Verfahren nach Anspruch 6, wobei das Ausgangsprozessfluid Diphenylmethandiisocyanat und Polymethylenpolyphenylenpolyisocyanate enthält und Diphenylmethandiisocyanat mindestens teilweise aus dem Film verdampft wird, während der Film durch das/die erwärmte(n) Hohlrohr(e) (4) abwärts fließt, um einen Diphenylmethandiisocyanat enthaltenden Dampfstrom und einen Flüssigkeitsstrom zu erzeugen, der, relativ zu dem Ausgangsprozessfluid mit Polymethylenpolyphenylenpolyisocyanaten angereichert und mit Diphenylmethandiisocyanat verarmt ist.

**Revendications**

1. Appareil à film tombant comprenant :

(a) une enveloppe externe (2) renfermant un volume intérieur,
(b) des plaques tubulaires supérieures (5) et inférieures (6) divisant le volume intérieur en chambres supérieure (26), médiane (3) et inférieure (7) distinctes,

(c) un ou plusieurs tubes creux orientés verticalement (4) ayant des extrémités supérieure et inférieure ouvertes, les tubes creux définissant chacun un chemin de fluide allant de la chambre supérieure (26) à la chambre inférieure (7),

(d) pour chaque tube creux (4) au moins unique, orienté verticalement, un insert de tube (12) associé localisé au niveau de l'extrémité supérieure du tube creux associé, l'insert de tube (12) comprenant un élément creux ayant (i) une section supérieure (25) se trouvant au-dessus de la plaque tubulaire supérieure (5), (ii) une section inférieure s'étendant dans le tube creux (4) associé, (iii) une ou plusieurs ouvertures de fluide de procédé (24) dans la section supérieure (25) destinées à l'admission d'un fluide de procédé dans l'insert de tube (12), (iv) une extrémité inférieure ouverte destiné au transfert d'un film mince du fluide de procédé à partir d'une surface intérieure de l'insert de tube (12) sur une surface intérieure du tube creux (4) associé ; et (v) une ou plusieurs nervures circonférentielles (22) se trouvant sur la surface intérieure de l'insert de tube (12) en dessous de la ou des ouverture(s) de fluide de procédé (24) ;

(e) au moins un orifice d'entrée de fluide de procédé (10) destiné à l'introduction d'un liquide de procédé dans la chambre supérieure (26) et en contact avec une surface supérieure de la plaque tubulaire supérieure (5),

(f) au moins un orifice de sortie de fluide de procédé (11) destiné au retrait de l'au moins un fluide de procédé traité de la chambre inférieure (7) ;

(g) au moins un orifice d'entrée de fluide d'échange thermique (8) destiné à l'introduction d'un fluide d'échange thermique dans la chambre médiane (3) ; et

(h) au moins un orifice de sortie de fluide d'échange thermique (9) destiné au retrait du fluide d'échange thermique de la chambre médiane (3).

2. Appareil à film tombant selon la revendication 1 dans lequel l'au moins un tube creux orienté verticalement (4) et les inserts de tube (12) associés ont chacun des coupes transversales circulaires, chaque nervure circonférentielle (22) a une largeur radiale $W_{Rib}$ d'au moins 0,25 mm et d'au plus $ID_I/8$, dans lequel $ID_I$ représente un diamètre interne de l'insert de tube (12), et chaque nervure circonférentielle (22) a des bords courbés vers l'intérieur (42) ayant un rayon de courbure $R_{Rib} < \delta_{Rib}/2$, dans lequel $\delta_{Rib}$ représente l'épaisseur longitudinale de la nervure circonférentielle (22).

3. Appareil à film tombant selon la revendication 1 ou 2 dans lequel la section supérieure de l'insert de tube (12) est conçue pour produire un écoulement tangentiel de fluide de procédé dans l'insert de tube (12).

4. Appareil à film tombant selon l'une quelconque revendication précédente dans lequel la ou les ouvertures de fluide de procédé (24) dans la section supérieure de la plaque tubulaire (12) sont des fentes hélicoïdales.

5. Appareil à film tombant selon l'une quelconque revendication précédente comprenant en outre un distributeur de fluide de procédé au-dessus de la plaque tubulaire supérieure.

6. Procédé destiné à la séparation de composants d'un mélange liquide, comprenant l'introduction d'un fluide de procédé contenant le mélange liquide dans un appareil à film tombant selon l'une quelconque revendication précédente, l'écoulement du fluide de procédé à travers le(s) insert(s) de tube (12) du film tombant pour former un film annulaire du fluide de procédé, puis le transfert du film du fluide de procédé sur des surfaces intérieures de tube(s) creux (4) chauffé(s) associé(s) de l'appareil à film tombant et l'écoulement du film vers le bas à travers le(s) tube(s) creux (4) chauffé(s), moyennant quoi au moins un composant du mélange liquide est au moins partiellement vaporisé du film à mesure que le film s'écoule vers le bas à travers le(s) tube(s) creux (4) chauffé(s), et au moins un composant du mélange liquide n'est pas volatilisé.

7. Procédé selon la revendication 6, dans lequel le fluide de procédé de départ contient du diisocyanate de diphénylméthane et des polyisocyanates de polyméthylène polyphénylène et le diisocyanate de diphénylméthane est au moins partiellement vaporisé du film à mesure que le film s'écoule vers le bas à travers le(s) tube(s) creux (4) chauffé(s) pour produire un courant de vapeur contenant du diisocyanate de diphénylméthane et un courant de liquide qui est, par rapport au fluide de procédé de départ, enrichi en polyisocyanates de polyméthylène polyphénylène et appauvri en diisocyanate de diphénylméthane.

# FIGURE 1

FIGURE 2

FIGURE 3

FIGURE 4

FIGURE 5

FIGURE 6

FIGURE 7

FIGURE 8

FIGURE 9

FIGURE 10

FIGURE 11

## FIGURE 12

## FIGURE 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4298041 A
- US 4154642 A
- US 4199537 A
- US 9101852 B
- US 20200030712 A